# EUROPÄISCHE PATENTSCHRIFT

(11) **EP 0 487 099 B1**
(45) Veröffentlichungstag und Bekanntmachung des Hinweises auf die Patenterteilung: **24.04.1996**
(21) Anmeldenummer: 91119946.1
(22) Anmeldetag: 22.11.1991
(51) Int. Cl.: C12N 15/31, C07K 14/215, C12N 1/21

(54) **Bakteriorhodopsin-Doppelmutanten**
Bacteriorhodopsine - double mutants
Bacteriorhodopsine - doubles-mutants

(30) Priorität: 23.11.1990 DE 4037342
(43) Veröffentlichungstag der Anmeldung: 27.05.1992
(73) Patentinhaber: Max-Planck-Gesellschaft zur Förderung der Wissenschaften e.V., D-37073 Göttingen (DE)
(72) Erfinder: Oesterhelt, Dieter, Prof. Dr., W-8000 München 70 (DE); von Gronau-Meessen, Susanne, D-8134 Pöcking (DE); Tittor, Jörg, W-8000 München 19 (DE); Matuszak, Anja, W-7700 Singen (DE); May, Klaus, W-8000 München 80 (DE)
(74) Vertreter: Schuderer, Michael

(56) Entgegenhaltungen:
- JOURNAL OF BIOLOGICAL CHEMISTRY, Band 264, Nr. 22, 05 August 1989, Baltimore, MD (US); J. SOPPA et al., Seiten 13049-13056
- GENE, Band 90, Nr. 1, 31 Mai 1990, Amsterdam (NL); BAOFU NI et al., Seiten 169-172
- PROCEEDINGS OF THE NATL. ACADEMY OF SCIENCES USA, Band 85, Nr. 12, Juni 1988, Washington, DC (US); TATSUSHI MOGI et al., Seiten 4148-4152
- BIOLOGICAL ABSTRACTS, Band BR40, Philadelphia, PA (US); J. TITTOR et al., Nr. 103892

## Beschreibung

Bakteriorhodopsin ist eine durch Licht aktivierbare Protonenpumpe, die in Halobakterien wie z.B. Halobacterium halobium vorkommt und aus einem Proteinanteil (Bakterioopsin) und einem Chromophor (Retinal) zusammengesetzt ist (siehe z.B. Oesterhelt und Tittor, TIBS 14 (1989), 57-61). Natives Bakteriorhodopsin, das aus dem Wildtyp von Halobacterium halobium erhältlich ist, besitzt ein Absorptionsmaximum bei einer Wellenlänge von 570 nm.

Das Absorptionsmaximum des Zwischenprodukts im angeregten Zustand hat eine Wellenlänge von 412 nm. Bakteriorhodopsin ist in zwei-dimensional kristallinen Bereichen der Zellmembran, der sog. Purpurmembran, organisiert. Bis zu 80% der Zellmembran von Halobacterium halobium können aus dieser Purpurmembran bestehen. Eine Isolierung des Bakteriorhodopsins in Form dieser Purpurmembran ist mit bekannten Mitteln möglich (Oesterhelt und Stoeckenius, Methods Enzymol. 31 Biomembranes, 667 - 678, 1974).

Neben dem Bakteriorhodopsin bilden Halobakterien Halorhodopsin, eine lichtgetriebene Chlorid-, Bromid-, Jodidpumpe. Dies ist in seiner Struktur dem Bakteriorhodopsin sehr ähnlich, wird jedoch nicht in Purpurmembran-ähnlichen Strukturen der Zellmembran gebildet. Der Gehalt an Halorhodopsin in der Zellmembran ist nur ein Zehntel dessen von Bakteriorhodopsin. Die Isolierung dieses Halorhodopsins ist aufgrund dieser Eigenschaften bedeutend aufwendiger. Wie Bakteriorhodopsin besitzt Halorhodopsin einen Photozyklus, das Absorptionsmaximum des Grundzustandes liegt bei einer Wellenlänge von 578 nm. Das langlebigste Zwischenprodukt besitzt ein Absorptionsmaximum von 520 nm.

Aufgrund der lichtelektrischen Eigenschaften von Bakteriorhodopsin und anderen Rhodopsinen wird angestrebt, diese Stoffe für Biocomputer und zur Mustererkennung zu verwenden (Biosystems 19 (1986), 223-236). Für diese Zwecke ist es wünschenswert, das Absorptionsmaximum des Bakteriorhodopsin und des Intermediats im Photozyklus zu längeren Wellenlängen hin zu verlagern, um die für die Anregung des Wildtyp Bakteriorhodopsin nötigen, teueren Speziallaser durch preisgünstige handelsübliche Laser ersetzen zu können.

Die Eigenschaft von Halorhodopsin, mit Hilfe von Lichtenergie Chloridionen pumpen zu können, wäre für eine Anwendung zur Entsalzung von Lösungen interessant. Bisher war die Gewinnung von Halorhodopsin so aufwendig, daß keine wirtschaftliche Nutzung möglich war. Deshalb ist es wünschenswert, Rhodopsine mit Eigenschaften des Halorhodopsins in Form von Purpurmembranen isolieren zu können.

Bisher sind mehrere Mutanten des Bakteriorhodopsins, bei denen sich ein Einfluß der Mutation auf den Photozyklus und die Absorptionsmaxima zeigte, bekannt.

Mogi et al (PNAS USA 85, 4148-4152, 1988) beschreiben Einzelaminosäure Mutanten, bei denen die Aminosäure Asp in den Positionen 85, 96, 115 und 212 gegen Glu bzw. Asn ausgetauscht ist. Bei weiteren Mutanten ist in Position 212 Asp gegen Asn, Glu oder Ala ersetzt. Die Expression der Proteine erfolgte in E. coli. Aus E. coli wurden die Proteine aufgereinigt. Durch Detergenz, Phospholipidbehandlung und Zugabe von Retinal wurden bakteriorhodopsinähnliche Chromophore regeneriert. Es wurde gezeigt, daß die Protonentranslokation vollkommen durch den Austausch Asp-85 zu Asn und teilweise (< 10%) durch den Austausch Asp-96 zu Asn und Asp-212 zu Glu aufgehoben wurde. Die Mutation Asp-85 zu Asn führte zu einer Verschiebung des Absorptionsmaximums zu λₘₐₓ = 590 nm.

Soppa et al (J. Biol. Chem. 264, 22, 13049-13056, (1989))beschreiben Bakteriorhodopsin Muteine mit folgenden Einzelaminosäuremutationen: Asp-85 zu Glu; Asp-96 zu Asn ; Asp-96 zu Gly. Diese Muteine wurden durch statistische Mutagenese mit Röntgen- oder UV-Strahlung erzeugt und durch Bromdeoxyuracil-Selektion photosynthetisch negativer Phänotypen selektiert. Sie wurden somit nicht zielgerichtet erzeugt. Im Unterschied zu den veränderten Proteinen, die von Mogi et al. untersucht wurden, wurden diese Muteine in Halobakterien exprimiert und in purpurmembranähnlichen Strukturen daraus isoliert.
Die Mutationen von Asp-96 verlangsamten die Kinetik des Photozyklus. Eine Veränderung der spektralen Eigenschaften des Bakteriorhodopsins trat hier nicht auf. Der Austausch der Aminosäure Asp-85 zu Glu führt zu einer Verschiebung des Absorptionsmaximums von 568 nm nach 610 nm. Die Deprotonierung der Aminosäure Glu-85 verschiebt das Absorptionsmaximum auf etwa 530 nm.

Subramaniam et al (Proc. Natl. Acad. Sci USA 87, 1013-1017 1990) untersuchen die Veränderung des Absorptionsspektrums von Bakteriorhodopsin durch Mutationen an Position Asp-85 und Arg-82. Der Austausch Asp-85 zu Asn ergibt einen blauen Chromophor (λₘₐₓ = 588 nm). Es ist keine Protonentranslokation mehr nachzuweisen. Untersuchungen zum Photozyklus wurden nicht gemacht.

Aufgabe der vorliegenden Erfindung war es, Bakteriorhodopsin Mutanten herzustellen, die sowohl ein in langwelligere Bereiche verschobenes Absorptionsmaximum wie auch einen Photozyklus besitzen, der ein langlebigstes Intermediat mit einem Absorptionsmaximum λ > 500 nm besitzt.

Eine weitere Aufgabe der Erfindung war es, ein Bakteriorhodopsin Mutein das Anionen pumpt, herzustellen.

Eine weitere Aufgabe war es, dieses Bakteriorhodopsin-Mutein effizient in biologisch aktiver Form zu exprimieren und in Form eines zweidimensionalen Lipidproteinkristalls "Purpurmembran" isolieren zu können.

Gegenstand der vorliegenden Erfindung sind Doppelmutanten des Bakteriorhodopsins, dadurch gekennzeichnet, daß die Aminosäurepositionen 85 und 96 des Opsins eines Halobacterien Wildtypstammes mutiert sind.

In einer bevorzugten Ausführungsform sind die Aminosäurepositionen 85 und 96 dieses Opsins von Asp zu Asn mutiert. (D85,96N)

In einer bevorzugten Ausführungsform stammt das Opsin aus dem Wildtypstamm von Halobacterium halobium.

Ein weiterer Gegenstand der Erfindung sind Mehrfachmutanten des Bakteriorhodopsins, dadurch gekennzeichnet, daß sie lichtgetrieben Anionen pumpen.

Die Erfindung beinhaltet weiterhin Mehrfachmutanten des Bakteriorhodopsins, dadurch gekennzeichnet, daß ihr Absorptionsmaximum größer als 578 nm ist.

Ein weiterer Gegenstand der Erfindung sind die "Purpurmembranen" aus den Halobacterienstämmen, die die erfindungsgemäßen Mutanten des Bakteriorhodopsins produzieren.

Die Erfindung beinhaltet weiterhin ein Verfahren zur Herstellung eines eine Mehrfachmutante des Bakteriorhodopsins produzierenden Halobakterienstammes, dadurch gekennzeichnet, daß man
1) ein Opsingen einer gerichteten Mutagenese unterzieht,
2) Zellen eines Stammes von Halobakterium bei dem das dem in Schritt 1) mutagenisierten Opsingen entsprechende Opsingen inaktiv ist oder dem das bop-Gen und andere Retinalproteingene fehlen, mit einem rekombinanten halobakteriellen Vektor transformiert, der mindestens eine Kopie des in Schritt 1 mutagenisierten Opsingens enthält und
3) die transformierten Halobacterium halobium Zellen regeneriert und kultiviert.

Die Gegenstände der Erfindung können zur optischen Informations-Verarbeitung mit holographischen Methoden eingesetzt werden. Beispiele sind Echtzeitinterferometrie, Mustererkennung und reversible holographische Informationsspeicherung. Eine weitere Einsatzmöglichkeit ist die Verwendung zur Entsalzung von salzhaltigen Lösungen.

Erfindungsgemäß wird ein bestimmtes Opsingen gezielt mutagenisiert. Die gezielte Mutagenese erfolgt außerhalb der H. halobium Zelle durch dem Fachmann auf dem Gebiet der Molekularbiologie an sich bekannte Methoden (z.B. Oligonukleotid gerichtete Mutagenese, chemische DNA-Synthese oder PCR-Reaktion). Unter Mutagenese ist der Austausch von 2 oder mehr Nukleotiden in einem Opsingen zu verstehen, die wiederum zu einem spezifischen Austausch von Aminosäureresten im resultierenden Genprodukt, dem Retinalprotein führen. Beim Bakteriorhodopsin erfolgt die Mutagenese bevorzugt von den Aminosäuren, die den Ionenleitkanal bilden oder bevorzugt von den Aminosäuren die an der Retinalbindung beteiligt sind und damit Farbe und Photozyklus beeinflussen. Besonders bevorzugt werden Mutationen in den Positionen 85 und 96 des Opsins eingeführt.

Erfindungswesentlich für die Expression der Muteine in Halobacterium ist, daß zur Transformation ein Halobacterium halobium Stamm verwendet wird, bei dem das, dem jeweiligen mutagenisierten Opsingen entsprechende Opsingen inaktiv ist, oder dem das bop-Gen und andere Retinalproteingene fehlen. Die Inaktivierung des Opsingens kann z.B. durch genetische Insertion, Deletion oder sonstige Veränderung erfolgt sein, so daß der Bakterienstamm nicht mehr in der Lage ist, ein funktionelles Opsin in der Zelle zu exprimieren. Die Zellen müssen jedoch in der Lage sein, Retinal zu synthetisieren, das sich in der transformierten Zelle als Pigment mit dem Opsin zum jeweiligen Retinalprotein verbinden kann. Ein geeigneter Bakterienstamm ist z.B. H. halobium IV-8, der eine ISH1-Insertion im Bakterioopsingen enthält (Betlach et al., Appl. Microbiol. 7 (1986), 83-89). Ebenfalls sehr gut geeignet ist der H. halobium Stamm L33 (DSM 5735), der eine ISH2-Insertion innerhalb des Bakterioopsingens enthält (DasSarma et al., Proc. Nat. Acad. Sci. USA 81 (1984), 125-129) oder HN5 (BR⁻ HR⁻ Insert)(DSM 6229). Der H. halobium Stamm L33 zeichnet sich dadurch aus, daß trotz der bekannten, extrem hohen Spontanmutationsraten bei H. halobium generell dieser Stamm seit Jahren sich als stabil herausgestellt hat. Wie in Stamm L33 ist auch in HN5 das Bakterioopsingen (bop) durch ein ca. 1 kb großes Insertionselement inaktiviert. Das Insertionselement befindet sich im Amino-terminalen Teil oder im Promotor-Bereich des bop-Gens. Zusätzlich befindet sich in HN5 jedoch ein ca. 1 kb großes Insertionselement im Bereich des hop-Gens, das für den Proteinanteil des Halorhodopsins kodiert. Durch diese Insertionen ist der Stamm HN5 phänotypisch Bakteriorhodopsin- und Halorhodopsin-negativ.

Zur Transformation von Halobakterien ist es zunächst erforderlich, Sphäroblasten herzustellen. Dies kann auf übliche Weise geschehen, wie z.B. bei Cline et al., (J. Bacteriol. 171 (1989), 4987-4991) oder bei Ni et al. (Gene 90 1990, 169-172) beschrieben.

Eine Transformation von H. halobium ist mit Bakteriophagen-DNA, Plasmid-DNA und linearer genomischer DNA möglich (Cline et al., Can. J. Microbiol. 35 (1989), 148-152). Somit können prinzipiell als rekombinante halobakterielle Vektoren für das erfindungsgemäße Verfahren Bakteriophagen, Plasmide oder lineare DNA-Moleküle verwendet werden. Zum Einbringen eines mutagenisierten Opsingens in H. halobium Sphäroblasten verwendet man vorzugsweise Plasmide. Zur Transformation von H. halobium prinzipiell geeignete Plasmidvektoren sind z.B. von Hackett und DasSarma (Can. J. Microbiol. 35 (1989), 86-91) beschrieben. In ein derartiges Plasmid kann auf übliche Weise ein Opsin-Gen, das bereits mutagenisiert sein kann, einkloniert werden. Soll ein Bakteriorhodopsin-Mutein exprimiert werden, so kann der Vektor ggf. neben dem Bakterioopsin-Gen (bop) zusätzlich das brp-Gen aus Halobacterium halobium enthalten. Weiterhin können zur Erhöhung der Expression des Genprodukts in der Umgebung des Strukturgens (z.B. Promotorbereich) Veränderungen eingeführt werden. Ein für das erfindungsgemäße Verfahren verwendeter halobakterieller Vektor enthält vorzugsweise einen Selektionsmarker, so daß eine bessere Selektion auf transformierte Zellen möglich ist. Ein für Halobakterien verwendbarer Selektionsmarker befindet sich z.B. auf dem Plasmid pH455 (Lam und Doolittle, Proc. Nat. Acad. Sci. USA 86 (1989), 5478-5482). Ein 15,8 kb HindIII-Fragment von Plasmid pH455 enthält ein Gen, das Resistenz gegen den HMG-CoA-Reduktase-Inhibitor Mevinolin (Monakolin K) (3α-Methylcompactin) in H. halobium und H. volcanii bewirkt. Besonders bevorzugt ist ein sogenannter Halobacterium-E. coli "Shuttle Vektor", der zusätzlich genetische Elemente enthält, die eine Vermehrung und Selektion in E. coli erlauben.

Die Anwesenheit eines halobakteriellen Selektionsmarkers auf dem Vektor ist bevorzugt, aber nicht unbedingt erforderlich, da die Selektion auf positiv transformierte Zellen auch durch die Farbveränderung der Zellen, bedingt durch die Expression des Retinalproteins, erfolgen kann.

In einer besonders bevorzugten Ausführungsform des erfindungsgemäßen Verfahrens dienen mutagenisierte Derivate des rekombinanten Plasmids p319 als halobakterieller Vektor. Die Details der Konstruktion eines mutagenisierten Derivats von p319 sind in den Beispielen und in den Figuren dargestellt.

Die Transformation von H. halobium mit Plasmid-DNA ist prinzipiell bekannt (Cline et al., Can. J. Microbiol. 35 (1989), 148-152). Ni et al. (Gene 90 (1990) 169-172).

In Anhang 1 ist die Nukleotidsequenz des mutagenen Oligonukleotids D85,96N wiedergegeben. Dargestellt ist die zur RNA komplementäre Sequenz. * kennzeichnet die Positionen in denen C gegen T ausgetauscht wurde.

In den Figuren werden folgende Abkürzungen verwendet:
- cat*:: inaktives Chloramphenicolacetyltransferase-Gen
- bla*:: inaktives Ampicillin-Resistenzgen (bla-Gen)
- Ap^{S}:: Ampicillin-sensitiv
- cat:: aktives Chloramphenicolacetyltransferase Gen
- bla:: aktives Ampicillin-Resistenzgen (bla-Gen)
- Cm^{R}:: Chloramphenicol resistent
- bop**:: mutiertes bop Gen D85,96N

Fig. 1 zeigt die Restriktionskarte des Plasmids Mc5-8/bop
Fig. 2 stellt die Mutagenese des nativen bop Gens schematisch dar
Fig. 3 zeigt die Konstruktion von pWT78
Fig. 4 zeigt die Konstruktion von p319/D85,96N

### Beispiele

Die nachfolgenden Beispiele sollen die Erfindung weiter verdeutlichen.

In-vitro Mutagenese des bop-Gens, Transformation eines bop-negativen Stammes von H. halobium mit einem Vektor, der das mutierte bop-Gen trägt, und Nachweis der Expression von verändertem Bakteriorhodopsin.

### 1. Mutagenese

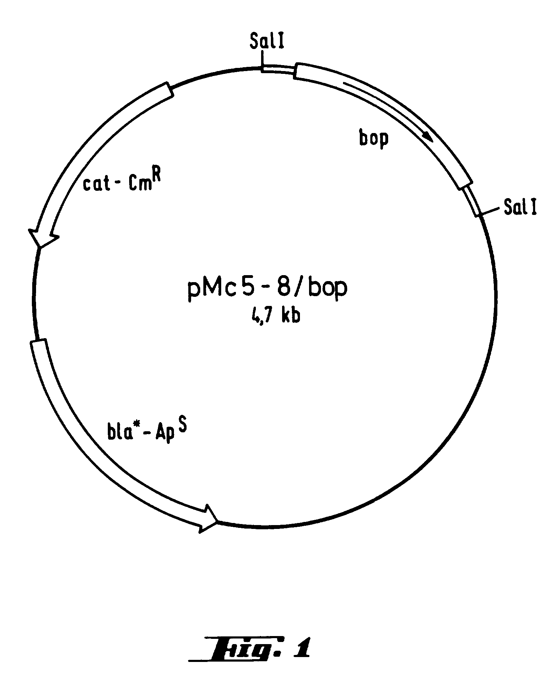

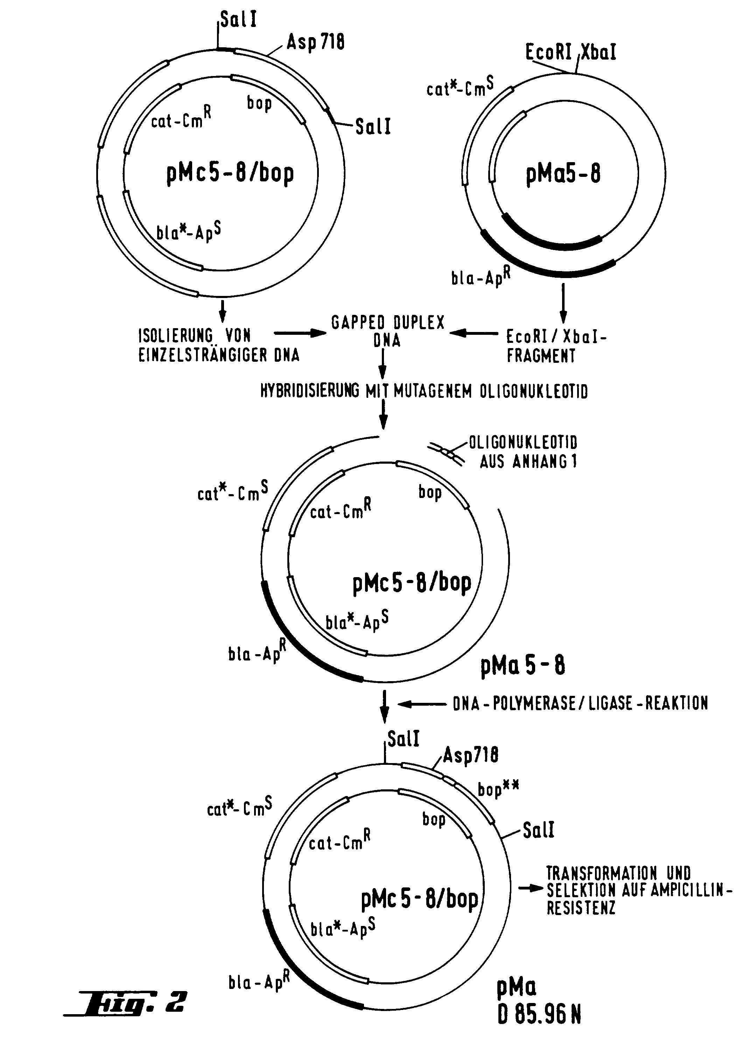

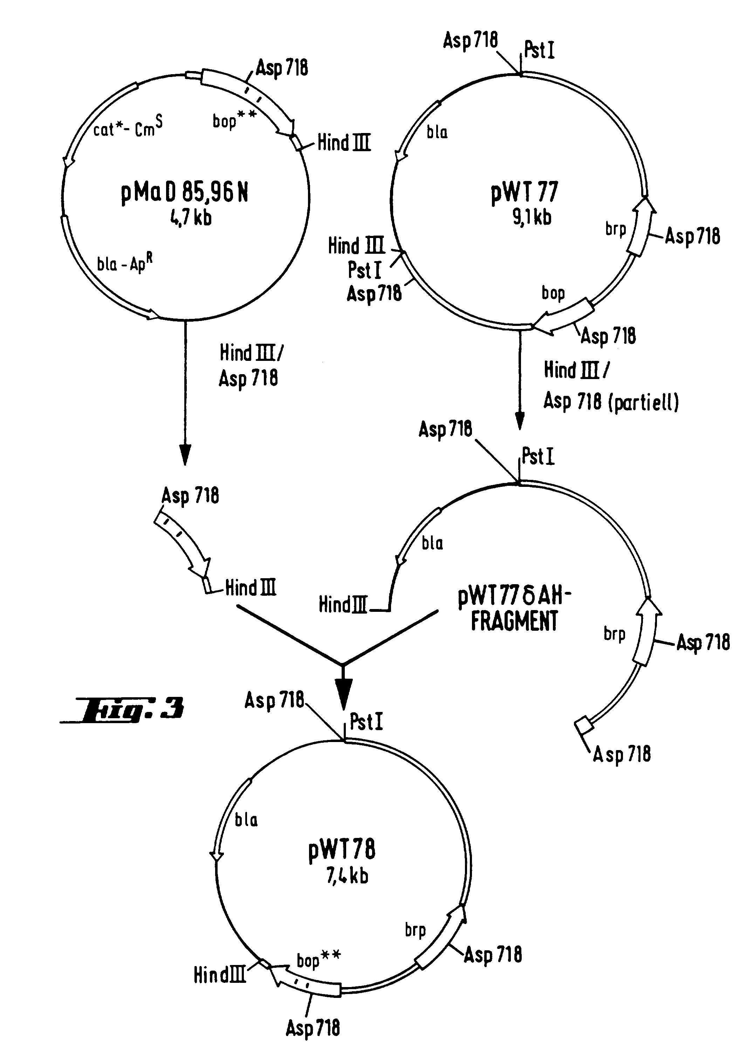

Das Plasmid pMc5-8/bop (Abb. 1) besteht aus einem 0.9 kb großen SalI-Fragment mit dem bop-Gen aus H. halobium, welches in den SalI geschnittenen Mutagenesevektor pMc5-8 (Stanssens et al. Nucleic Acids Research 17, 4441-4454, 1989) kloniert wurde. Das erhaltene Plasmid pMc5-8/bop (Abb. 1) besitzt ein inaktives Ampicillin-Resistenzgen (bla*) sowie ein aktives Chloramphenicolacetyltransferase-Gen (cat). pMc 5-8/bop wurde nach der von Stanssens et al. (Nucleic Acids Research 17, 4441-4454, 1989) beschriebenen Methode mutagenisiert (Abb. 2). Dazu wurde einzelsträngige DNA von pMc5-8/bop isoliert und mit dem EcoRI/XbaI-Fragment von pMa5-8 hybridisiert. Das Plasmid pMa 5-8 trägt ein inaktives Chloramphenicolacetyltransferase-Gen (cat*) sowie ein aktives Ampicillin-Resistenzgen (bla). Zur Herstellung der gapped duplex DNA wurde ein Gemisch, bestehend aus dem pMa5-8-Fragment (0.1 pmol) und der einzelsträngigen DNA (0.5 pmol) in einem Volumen von 36 µl bei 70°C für 5 min inkubiert und dann nach Zugabe von 4 µl Hybridisierungspuffer (1.5 M KCl, 100 mM Tris/Cl pH 7.5) - erhitzt auf 70°C - auf Raumtemperatur abgekühlt. 8 µl dieses Ansatzes wurden dann mit 4 bis 10 pmol mutagenem Oligonukleotid (2 µl) vermischt, zur Hybridisierung für 5 min auf 65°C erhitzt, und auf Raumtemperatur abgekühlt. Das zur Mutagenese eingesetzte 70 mere Oligonukleotid kodierte anstelle von Asp an Position 85 und 96 im bop-Gen jeweils für Asn (Anhang 1). Zur DNA-Polymerase/Ligase-Reaktion wurde der Ansatz mit 4 µl 10fach Puffer (625 mM KCl, 275 mM Tris-HCl, 150 mM MgCl₂, 20 mM DTT, 0.5 mM ATP, je 0.25 mM der vier dNTP's, pH 7.5) versetzt, auf 40 µl mit H₂O aufgefüllt, und nach Zugabe von 1 Unit DNA-Polymerase (Klenow-Fragment) und 5 Units T4-DNA-nach Zugabe von 1 Unit DNA-Polymerase (Klenow-Fragment) und 5 Units T4-DNA-Ligase bei Raumtemperatur für 45 min inkubiert. Nach Transformation des DNA-Polymerase/Ligase-Ansatzes wurde mit Hilfe der unterschiedlichen Resistenzmarker der beiden Stränge auf den mutierten Strang (Ampicillin-Resistenz) selektiert. Dadurch wurde das Plasmid pMaD85,96N erhalten (Abb. 2, Abb. 3). Dies Plasmid enthält das in Position 85 und 96 mutierte bop-Gen.

### 2. Umklonierung des mutierten bop-Gens für die Expression in Halobacterium halobium

### a) E. coli-Vektor

Abb. 3 beschreibt die Konstruktion von pWT78 aus den Plasmiden pMaD85,96N und pWT77. Das Plasmid pWT77 ist das kommerziell erhältliche E. coli Plasmid pGEM4, in dessen PstI-Restriktionsstelle ein halobakterielles PstI-Fragment mit dem Wildtyp bop-Gen und brp-Gen einkloniert wurde. pWT77 wurde mit HindIII linearisiert und mit Asp718 partiell gespalten. Das 6.8 kb HindIII/Asp718-Fragment WT77δAH mit dem Vektoranteil (pGEM4) und dem 5'-Bereich des Wildtyp bop-Gens wurde mit dem HindIII/Asp718-Fragment aus pMaD85,96N, welches den mutierten 3'-Bereich des bop-Gens enthält, ligiert. Dabei entsteht das 7.4 kb große Plasmid pWT78.

### b) E. coli-Halobacterium-Vektor

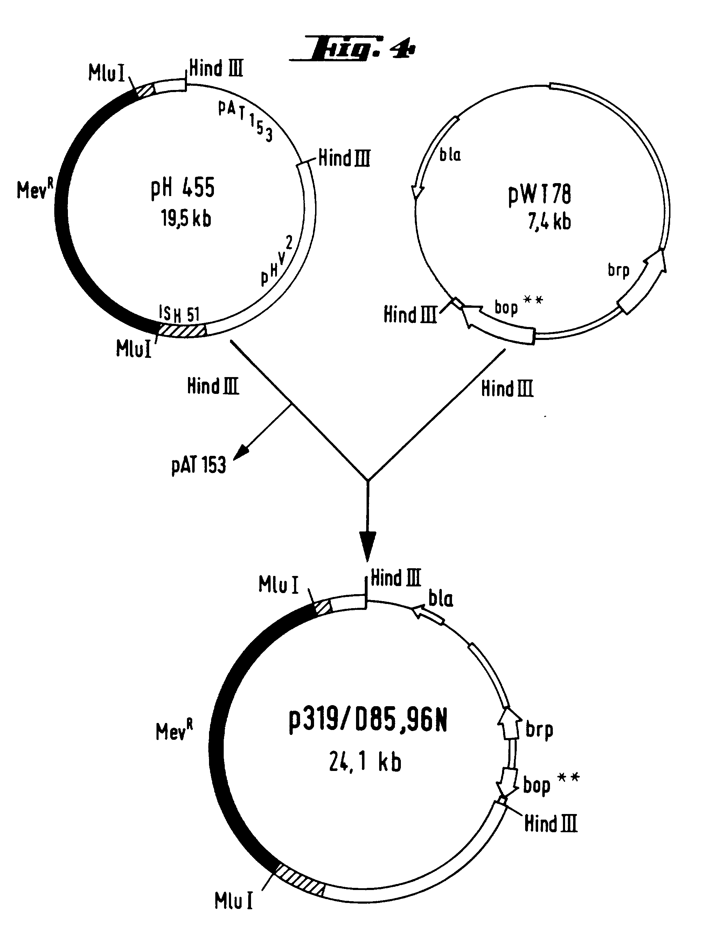

Zur Expression des mutierten bop-Gens in Halobacterium halobium wurde das Plasmid p319/D85,96N verwendet. Die Konstruktion ist in Abb. 4 gezeigt, sie ist analog zu der von Ni et al. beschriebenen Konstruktion des Plasmids p319 (Ni et al., Gene 90 (1990) 169-172). Das Plasmid pH455 wurde von Lam und Doolittle (Proc. Natl. Acad. Sci. USA 86, (1989) 5478-5482) beschrieben. Dieses Plasmid enthält neben E. coli Anteilen (pAT153) das Mevinolin-Resistenzgen ISH51-DNA und das endogene Halobacterium volcanii Plasmid pHV2. pH455 wurde mit HindIII gespalten und das Fragment mit ca. 16kb, welches das Mevinolin-Resistenzgen und den pHV2-Anteil enthält, wurde mit dem HindIII-linearisierten Plasmid pWT78 ligiert. Das erhaltene Plasmid p319/D85,96N (DSM 6225) wurde für die Transformation von Halobacterium halobium L33 verwendet.

### 3. Transformation und Expression

Für die Transformation wurde der Stamm Halobacterium halobium L33 (DSM 5735) verwendet. Die Transformation wurde nach der von Ni et al., Gene 90 (1990) 169-172 entwickelten Methode durchgeführt.

### a) Sphäroblastenbildung

1 ml einer 35 ml Zellkultur (ca. 90 Kletteinheiten) von Halobacterium halobium L33 (DSM 5735) in Wachstumsmedium (250 g NaCl, 20 g MgSO₄ · 7H₂O, 3 g Trinatriumcitrat · 2H₂O, 2 g KCl, 0.2 g CaCl₂ · 2H₂O, 25 ml 2 mol/l Tris-HCl pH 7.2, 3 g Yeastextrakt (Difco), 5 g Trypton (Difco) und H₂O ad 1000 ml) wird in 1.5 ml Eppendorf-Gefäßen 15 Minuten lang bei 3000 bis 4000 Upm bei Raumtemperatur abzentrifugiert. Der Überstand wird mit einer Pasteur-Pipette möglichst vollständig abgenommen. Die Zellen werden in 100 µl sphäroblastenbildender Lösung (SPH: 2 mol/l NaCl, 25 mmol/l KCl, 50 mmol/l Tris-HCl pH 8,75, 15% Saccharose, steril filtriert) aufgenommen. Anschließend werden 10 µl 0.5 mol/l EDTA in SPH auf den oberen inneren Rand des Gefäßes pipettiert und durch schnelles Mischen (Schwenken des Eppendorfgefäßes) mit der Zellsuspension vereinigt.

Die Bildung der Sphäroblasten erfolgt bei Raumtemperatur innerhalb von ca. 30 Minuten, unter einem Lichtmikroskop kann die Vollständigkeit der Sphäroblastenbildung kontrolliert werden.

### b) Transformation der Sphäroblasten

Die DNA wird in einem Volumen von 20 µl (bestehend aus 15 µl DNA-Lösung (10 µg in 15 µl 50 mM Tris Cl, pH 7.5, 1 mM EDTA) und 5 µl 0,5 mol/l EDTA in SPH) zu der Sphäroblastensuspension gegeben und 5 Minuten lang bei Raumtemperatur inkubiert. Pro Transformationsansatz wurden zwischen 3 und 10 µg Plasmid-DNA eingesetzt. Die Polyethylenglykol-Lösung (240 µl) wurde in den Deckel des Eppendorfgefäßes gegeben und durch schnelles Mischen mit der SPH-Suspension vereinigt. Die Inkubation beträgt 20 Minuten bei Raumtemperatur.

Die Polyethylenglykol-Lösung besteht aus: 60% (v/v) PEG 600 (Sigma) und 40% SPH. Das verwendete PEG 600 wurde in einigen Fällen wie folgt gereinigt.

Die Reinigung von PEG 600 lehnt sich an die Vorschrift von Klebe et al. Gene 25 (1983) 333 ff an. 30 g PEG 600 wurden in 30 ml Benzol gelöst. Zu dem PEG 600/Benzol-Gemisch wurden 60 ml Isooctan hinzugefügt und 30 min auf Eis gestellt. Das PEG 600 verfestigte sich und man konnte danach die obere organische Phase abdekantieren. Das PEG 600 wurde im Wasserbad auf Raumtemperatur erwärmt. Wieder wurden 60 ml Isooctan zum PEG 600 hinzugefügt, gut geschüttelt und das Gemisch über Nacht bei Raumtemperatur stehen gelassen. Die Isooctanphase wurde verworfen und das PEG 600 im Scheidetrichter mit 2mal 150 ml Diethylether ausgeschüttelt. Der Diethylether wurde dann am Rotationsverdampfer entfernt (ca. 2 h).

### c) Regeneration der transformierten Sphäroblasten

Der Transformationsansatz wird mit 1 ml Regenerationslösung (REG: 3,5 mol/l NaCl, 150 mmol/l MgSO₄ · 7H₂O, 50 mmol/l KCl, 7 mmol/l CaCl₂ · 2H₂O, 50 mmol/l Tris-HCl, pH 7,2, 15% Saccharose steril filtrieren) verdünnt, gemischt und wie oben angegeben, abzentrifugiert. Dann wird der Überstand mit einer ausgezogenen Pasteur-Pipette vollständig abgenommen, das Bakterienpellet (häufig nicht sichtbar) in 1 ml Vollmedium (Mischung aus 80 ml H₂O mit 250 g/l NaCl, 20 g/l MgSO₄ · 7H₂O, 3 g/l Trinatriumcitrat · 2H₂O, 2 g/l KCl, 0,2 g/l CaCl₂ · 2H₂O und 30 ml H₂O mit 18 g Saccharose, 0,6 g Trypton, 0,4 g Yeast-Extrakt steril filtrieren) vorsichtig suspendiert und bei 37°C über Nacht inkubiert.

### d) Ausplattierung

Jeweils 0,1 bis 0,25 ml der Zellen werden in 3 ml frisch hergestelltem Overlay-Medium (25 g NaCl, 2 g MgSO₄ · 7H₂O, 0,3 g Trinatriumcitrat · 2H₂O, 0,2 g KCl, 0.02 g CaCl₂ · 2H₂O auf 85 ml mit H₂O auffüllen und pH 7.2 einstellen; 0,3 g Hefeextrakt, 0,5 g Trypton, 0,8 g Agar auf 15 ml auffüllen; beide Komponenten autoklavieren, zusammengeben und 15 g Saccharose/100 ml Lösung sowie Mevinolin bis zu einer Endkonzentration von 20 µmol/l bei 56°C hinzufügen) bei etwa 45°C aufgenommen. Diese Suspension wird sofort auf Support-Platten (250 g NaCl, 20 g MgSO₄ · 7H₂O, 3 g Trinatriumcitrat · 2H₂O, 2 g KCl auf 850 ml auffüllen, 3 g Yeast-Extrakt, 5 g Trypton, 1,5% Agar auf 350 ml auffüllen, beides autoklavieren, nach dem Autoklavieren zusammengeben und Mevinolin bis zu einer Endkonzentration von 20 µmol/l hinzufügen) gegossen.

Die Platten werden versiegelt und für mindestens 4 Wochen bei 40°C im Brutschrank belassen.

### 4. Anzucht der Transformanten und Isolierung des mutierten Bakteriorhodopsins

Kolonien transformierter Zellen, die oft erst nach Wochen erscheinen, sind im Gegensatz zu den Kolonien spontan mevinolin-resistenter Zellen (gelblich) grünlich gefärbt und werden in 35 ml Vollmedium, das 20 µmol/l Mevinolin enthält, vermehrt. Aus der Zellmischung können durch Klonieren homologe Rekombinanten isoliert werden, die den Zusatz von Mevinolin zum Medium unnötig machen.

Die Isolierung des BR Muteins mit den Aminosäureaustauschen von Asp zu Asn an Pos. 85 und 96 (D85,96N) erfolgt durch Saccharosedichtegradientenzentrifugation wie in Oesterhelt und Stoeckenius (Methods Enzymol. 31, Biomembranes, 667-678, (1974)) beschrieben. Das Protein wird als blaue Membran einer Schwimmdichte wie die Purpurmembran des Wildtyps erhalten.

### 5. Charakterisierung der Bakteriorhodopsinmutante

Die Membranfraktion, die als einziges Protein das BR Mutein D85,96N enthält, hat die Schwimmdichte von etwa 1.2 g/cm³, die derjenigen der Purpurmembran entspricht. Das Absorptionsmaximum des Proteins ist bei 608 nm (10 mM Tris-Puffer pH 7.0). Der pK Wert der Schiffbase des Retinals ist gegenüber dem Wildtyp von 13 auf 8.5 in Salzkonzentrationen über 1 M NaCl gesenkt und entspricht ebenso wie das Fehlen eines M-Intermediates im Photozyklus dem Verhalten von Halorhodopsin. Statt des M-Intermediates mit einer deprotonierten Schiffbase tritt als maximal blau verschobenes Intermediat eine Spezies auf, die nach photometrischen Bestimmungen ein Differenzmaximum bei 535 nm hat. Der Ausgangszustand wird mit 5 ms repopuliert.

Die BR Mutein D85,96N-haltige Membran wurde an schwarze Filmmembranen adsorbiert und nach der in Bamberg et al. (Biochim. Biophys. Acta 773,53-60, 1984) beschriebenen Methode untersucht. Die dabei gemessenen chloridabhängigen, lichtinduzierten stationären Ströme belegen, daß das Mutein nicht nur die photochemischen Eigenschaften des Halorhodopsins, sondern auch dessen Ionentranslokationsaktivität besitzt.

## Patentansprüche

1. Mutante des Bakteriorhodopsins, dadurch gekennzeichnet, daß in den Positionen 85 und 96 des Opsins eines Halobakterien Wildtypstammes Asparaginsäure zu Asparagin mutiert ist.

2. Mutante gemäß Anspruch 1, dadurch gekennzeichnet, daß das Opsin aus dem Wildtypstamm vom Halobacterium halobium stammt.

3. Mutante des Bakteriorhodopsins gemäß Anspruch 1 oder 2, dadurch gekennzeichnet, daß ihr Absorptionsmaximum größer als 578 nm ist und das langlebigste Zwischenprodukt im Photozyklus ein Absorptionsmaximum bei einer Wellenlänge größer als 500 nm hat.

4. Mutante des Bakteriorhodopsins gemäß Anspruch 3, dadurch gekennzeichnet, daß ihr Absorptionsmaximum größer als 578 nm ist und das langlebigste Zwischenprodukt im Photozyklus ein Absorptionsmaximum bei einer Wellenlänge von 520 bis 540 nm hat.

5. Mutante des Bakteriorhodopsins gemäß einem oder mehreren der Ansprüche 1-4, dadurch gekennzeichnet, daß sie lichtgetrieben Anionen pumpen.

6. Halobakterienstämme, dadurch gekennzeichnet, daß sie ein Bakteriorhodopsin nach einem der Ansprüche 1 bis 5 produzieren.

7. Zellmembranfragmente enthaltend die Muteine aus Halobakterienstämmen nach Anspruch 6.

8. Verfahren zur Herstellung eines Halobakterienstammes nach Anspruch 6 dadurch gekennzeichnet, daß man
1) ein Opsingen einer gerichteten Mutagenese unterzieht,
2) Zellen eines Stammes von Halobakterium halobium, bei dem das dem in Schritt 1) mutagenisierten Opsingen entsprechende Opsingen inaktiv ist oder dem das bop Gen und andere Retinalproteingene fehlen, mit einem rekombinanten Halobakterium Vektor transformiert, der mindestens eine Kopie des in Schritt 1 mutagenisierten Opsingens enthält und
3) die transformierten Halobakterium halobium Zellen regeneriert und kultiviert.

9. Verfahren nach Anspruch 8, dadurch gekennzeichnet, daß man das Plasmid p319/D85,96N (DSM 6225) oder ein mutagenisiertes Derivat davon als rekombinanten Vektor verwendet.

10. Vektor p319/D85,96N (DSM 6225).

11. H.halobium Stamm HN5 (DSM 6229).

## Claims

1. Mutant of bacteriorhodopsin, characterized in that aspartic acid is mutated to asparagine in positions 85 and 96 of the opsin of a wild-type strain of Halobacteria.

2. Mutant according to Claim 1, characterized in that the opsin originates from the wild-type strain of Halobacterium halobium.

3. Mutant of bacteriorhodopsin according to Claim 1 or 2, characterized in that its absorption maximum is above 578 nm, and the intermediate with the longest life in the photocycle has an absorption maximum at a wavelength above 500 nm.

4. Mutant of bacteriorhodopsin, according to Claim 3, characterized in that its absorption maximum is above 578 nm, and the intermediate with the longest life in the photocycle has an absorption maximum at a wavelength of from 520 to 540 nm.

5. Mutant of bacteriorhodopsin according to one or more of Claims 1-4, characterized in that they [sic] carry out light-driven pumping of anions.

6. Strains of Halobacteria characterized in that they produce a bacteriorhodopsin according to any of Claims 1 to 5.

7. Cell membrane fragment containing the muteins from strains of Halobacteria according to Claim 6.

8. Process for preparing a strain of Halobacteria according to Claim 6, characterized in that
1) an opsin gene is subjected to directed mutagenesis,
2) cells of a strain of Halobacterium halobium in which the opsin gene corresponding to the opsin gene mutated in step 1) is inactive, or which lacks the bop gene and other retinal protein genes, are transformed with a recombinant Halobacterium vector which contains at least one copy of the opsin gene mutated in step 1, and
3) the transformed Halobacterium halobium cells are regenerated and cultivated.

9. Process for preparing a strain of Halobacteria according to Claim 8, characterized in that the plasmid p319/D85,96N (DSM 6225) or a mutated derivative thereof is used as recombinant vector.

10. Vector p319/D85,96N (DSM 6225).

11. H.halobium strain HN5 (DSM 6229).

## Revendications

1. Mutants de la bactériorhodopsine, caractérisés en ce qu'un résidu acide aspartique est muté en asparagine aux positions 85 et 96 de l'opsine d'une souche de type sauvage d'halobactérie.

2. Mutants selon la revendication 1, caractérisés en ce que l'opsine provient de la souche de type sauvage de *Halobacterium balobium.*

3. Mutants de la bactériorhodopsine selon la revendication 1 ou 2, caractérisés en ce que leur maximum d'absorption est supérieur à 578 nm et le produit intermédiaire ayant la plus longue durée de vie dans le photocycle présente un maximum d'absorption à une longueur d'onde supérieure à 500 nm.

4. Mutants de la bactériorhodopsine selon la revendication 3, caractérisés en ce que leur maximum d'absorption est supérieur à 578 nm et le produit intermédiaire ayant la plus longue durée de vie dans le photocycle présente un maximum d'absorption à une longueur d'onde de 520 à 540 nm.

5. Mutants de la bactériorhodopsine selon une ou plusieurs des revendications 1 à 4, caractérisés en ce qu'ils pompent des anions actionnés par la lumière.

6. Souches d'halobactéries, caractérisées en ce qu'elles produisent une bactériorhodopsine selon l'une des revendications 1 à 5.

7. Fragments de membrane cellulaire, contenant les mutéines provenant de souches d'halobactéries selon la revendication 6.

8. Procédé pour la production d'une souche d'halobactérie selon la revendication 6, caractérisé en ce que
1) on soumet un gène d'opsine à une mutagénèse dirigée,
2) des cellules d'une souche *d'Halobacterium halobium,* dans laquelle le gène d'opsine correspondant au gène d'opsine soumis à la mutagénèse dans l'étape 1) est inactif, ou qui est privée du gène bop et d'autres gênes de protéines rétiniennes, sont transformées par un vecteur recombinant *d'Halobacterium* qui contient au moins une copie du gène d'opsine soumis à la mutagénèse dans l'étape 1, et
3) les cellules *d'Halobacterium halobium* transformées sont régénérées et cultivées.

9. Procédé pour la production d'une souche d'halobactérie selon la revendication 8, caractérisé en ce que, comme vecteur recombinant, on utilise le plasmide p319/D85,96N (DSM 6225) ou un dérivé de celui-ci soumis à une mutagénèse.

10. Vecteur p319/D85,96N (DSM 6225).

11. Souche de *H. Halobium* HN5 (DSM 6229).
